# EUROPÄISCHE PATENTSCHRIFT

(11) **EP 3 636 302 B1**
(45) Veröffentlichungstag und Bekanntmachung des Hinweises auf die Patenterteilung: **16.04.2025**
(21) Anmeldenummer: 19202172.3
(22) Anmeldetag: 09.10.2019
(51) Int. Cl.: A61M 5/28, A61M 5/31

(54) **SPRITZENKÖRPER**
SYRINGE BODY
CORPS DE SERINGUE

(30) Priorität: 11.10.2018 DE 202018105835 U
(43) Veröffentlichungstag der Anmeldung: 15.04.2020
(73) Patentinhaber: SCHOTT AG, 55122 Mainz (DE)
(72) Erfinder: KÜCÜK, Mustafa, 9422 Staad (CH)
(74) Vertreter: Blumbach · Zinngrebe Patentanwälte PartG mbB

(56) Entgegenhaltungen:
- EP-B1- 0 720 857
- US-A1- 2009 171 297
- US-A1- 2012 041 388
- US-A1- 2013 178 737

## Beschreibung

Die Erfindung betrifft einen Spritzenkörper für eine pharmazeutische oder kosmetische Spritze, umfassend einen zylindrischen Hohlraum mit einer ersten Grundfläche mit einem Konus, einer zweiten, offenen Grundfläche zum Einführen eines Kolbens, und mit einem im Bereich des Umfangs der offenen Grundfläche angeordneten Fingerflansch, wobei der Fingerflansch auf der dem Konus zugewandten Seite ein Antirutsch-Element zum Verhindern des Abrutschens eines Fingers beim Injektionsvorgang aufweist sowie ein Verfahren zum Applizieren eines Pharmazeutikums oder Kosmetikums.

Als Spritze bezeichnet man ein medizinisches Instrument, das zur Verabreichung (Injektion) von flüssigen oder fluiden Medikamenten oder Kosmetika- sogenannten Injektabilia - verwendet wird, welche allgemein im Rahmen der vorliegenden Offenbarung auch abkürzend als Fluide bezeichnet werden. Eine Spritze umfasst in der Regel einen Spritzenkörper mit einem zylindrischen Hohlraum und einem darin bewegten Kolben. Dabei ist an einer Grundfläche des Zylinders in der Regel ein Konus, ggf. auch mit Schraubgewinde angeordnet, an den eine Kanüle oder ein Schlauch angeschlossen werden. Auf der gegenüberliegenden Seite, d.h. der anderen Grundfläche des Zylinders ist dieser offen, so dass auf dieser Seite der Kolben eingeführt wird, mit dem die Spritze sowohl aufgezogen werden kann, d.h. durch Herausziehen des Kolbens ein Fluides Pharmazeutikum oder Kosmetikung durch den Konus eingesaugt werden kann, als auch das Pharmazeutikum oder Kosmetikung verabreicht werden kann.

Alternativ kann die Spritze bereits mit einem Pharmazeutikum oder Kosmetikum bereits herstellerseitig, insbesondere steril befüllt sein, sodass es eines Aufziehens des Pharmazeutikums oder Kosmetikums vor dessen Applikation nicht bedarf.

Ferner können die vorliegend offenbarten Spritzenkörper und diese umfassenden Spritzen sowohl im human- als auch im veterinärmedizinischen Bereich Anwendung finden.

Zum Verabreichen des fluiden Pharmazeutikums oder Kosmetikums aus dem zylindrischen Hohlkörper wird der Kolben hineingedrückt, so dass der Inhalt durch den Konus gepresst wird. Damit dieser Vorgang mit einer Hand durchgeführt werden kann, ist üblicherweise im Bereich des Umfangs der offenen Grundfläche, d.h. um die Öffnung, in die der Kolben eingeführt ist, ein Fingerflansch angeordnet. Dieser bildet eine Griffmöglichkeit, um den Spritzenkörper gegen den Druck auf den Kolben zu halten. Üblicherweise wird der Spritzenkörper mit Zeige- und Mittelfinger am Fingerflansch gehalten und mit dem Daumen der Kolben in den Spritzenkörper gedrückt.

Um dabei ein Abrutschen eines Fingers beim Injektionsvorgang zu verhindern, ist insbesondere bei größeren Spritzen ab etwa 20 ml häufig an der Auflagefläche der Finger, d.h. auf der dem Konus zugewandten Seite des Fingerflansches ein Antirutsch-Element angeordnet. Häufig sind hierzu erhabene Querstreben vorgesehen, die tangential beabstandet ausgerichtet sind.

Das Dokument US 4 994 012 B zeigt einen entsprechenden Spritzenkörper, welcher zur Strahlungsabschirmung Wolfram aufweist, sowie eine diesen aufweisende Spritze.

In US 6 004 299 B wird eine Spritze mit einem aus Glas bestehenden Spritzenkörper offenbart, der seitliche Fingerauflageflächen definiert, welche scheinbar Längsrippen ausbilden.

US 2012/0041388 A zeigt eine Greifvorrichtung für einen Spritzenkörper, welche bis auf den Daumen nahezu vollständig mit den Fingern einer Hand umschlossen werden kann. Durch diese prinzipbedingte Größe der Greifvorrichtung, in welche der Spritzenkörper einzulegen ist, wird jedoch der jeweilige Spritzenkörper weniger gut handhabbar, da nicht mehr die feinfühligere Bewegung der jeweiligen die Spritze betätigenden Finger zur Verfügung steht, sondern lediglich nur noch Bewegungen der gesamten Hand zur Verfügung stehen. Ferner erscheint es als umständlich die Greifvorrichtung zunächst an dem Spritzenkörper anzubringen, um danach die Spritze verwenden zu können.

Auch das Dokument US 2013/0178737 A zeigt eine Greifvorrichtung für einen Spritzenkörper, bei welcher es als umständlich erscheint, die Greifvorrichtung zunächst an dem Spritzenkörper anzubringen, um danach die Spritze verwenden zu können.

Die US-amerikanische Schutzrechtsanmeldung US 2009/171297 A1 beschreibt einen einstückigen Spritzenkörper mit Fingerflansch, welcher aber kein Antirutsch-Element umfasst.

Es hat sich herausgestellt, dass beispielsweise Längsrippen aufweisende Konstruktionen zu Lagerungsproblemen der Spritzenkörper führen können:
Nach dem oben beschriebenen Prinzip gegossene Spritzenkörper neigen zum Verkippen, wenn man sie an der Flanschfläche auf eine plane Unterlage stellt.

Es ist daher Aufgabe der Erfindung, einen Spritzenkörper der eingangs genannten Art anzugeben, der besonders stabil zu lagern ist.

Diese Aufgabe wird erfindungsgemäß mit einem Spritzenkörper nach Anspruch 1 gelöst.

Die Erfindung geht dabei von der Überlegung aus, dass eine besonders stabile Lagerung dadurch erreichbar wäre, dass ein Verkippen des Spritzenkörpers beim Stellen auf die Flanschfläche vermieden wird. Hierbei wurde erkannt, dass das Verkippen auf eine unregelmäßige Oberfläche zurückzuführen ist, die offenbar beim Guss des Flansches entsteht. Insbesondere wurde erkannt, dass Einfallstellen in der Art von Einsackungen entstehen, die in ihrer Ausdehnung den eingangs beschriebenen Querstreben entsprechen. Erhabene Strukturen zur Erhöhung der Griffigkeit führen demnach zu Einfallstellen auf der gegenüberliegenden Seite und damit zu einer unregelmäßigen Oberfläche. Basierend auf der weiteren Erkenntnis, dass diese Einfallstellen durch eine lokale Materialanhäufung entstehen, die durch die Form der Querstreben entstehen, sollte eine derartige Materialanhäufung vermieden werden. Um aber dennoch die Griffigkeit und Abrutschfestigkeit zu erhalten, sollte eine alternative, nicht zu lokalen Materialanhäufungen führende Ausgestaltung des Antirutsch-Elements vorgesehen werden.

Hierfür sollte eine gleichmäßig aufgeraute Oberfläche verwendet werden, die lokale Materialanhäufungen vermeidet. Damit jedoch die Griffigkeit gewährleistet ist, sollte eine entsprechend hohe Rauheit der Oberfläche gewährleistet sein.

In Versuchen hat sich ein Mittenrauwert von mehr als 5 µm als geeignet erwiesen, um die gewünschte Abrutschsicherheit zu gewährleisten.

Nach der Erfindung ist dabei das Antirutsch-Element als Oberfläche mit einem arithmetischen Mittenrauwert zwischen 5 und 50 µm ausgebildet.

Dabei bildet der Fingerflansch mit der offenen Grundfläche eine Ebene, d.h. die Öffnung zum Einführen des Kolbens der Spritze liegt im ebenen Fingerflansch. Wie oben beschrieben ist gerade bei derartigen Spritzenkörpern das Problem des Verkippens beim Stellen auf die Ebene des Flansches gegeben, so dass die oben beschriebene Ausgestaltung des Antirutsch-Elements besondere Vorteile bietet.

Weiterhin ist der Flansch vorteilhafterweise bezüglich einer die Achse des zylindrischen Hohlraums enthaltenden Fläche spiegelsymmetrisch ausgebildet. Ein derartiger Flansch weist also zwei symmetrische Erstreckungen beidseits der Öffnung für den Kolben auf, so dass für Zeige- und Mittelfinger eine geeignete Auflagefläche zur Verfügung steht. Entsprechend ist auf beiden Auflageflächen ein Antirutsch-Element vorgesehen.

In weiterer vorteilhafter Ausgestaltung ist auf der dem Konus abgewandten Seite des Fingerflansches eine Beschriftungsfläche angeordnet. In einer derartigen Ausgestaltung bietet die oben beschriebene Ausgestaltung des Antirutsch-Elements zusätzliche Vorteile: In der Regel ist der Spritzenkörper aus einem durchsichtigen Material gefertigt. Dadurch scheint das Antirutsch-Element auf der Rückseite der Beschriftungsfläche durch. Die eingangs beschriebenen Querstreben verschlechtern hierbei die Lesbarkeit der Beschriftung durch Brechungs- und Spiegelungseffekte. Eine raue Oberfläche hingegen - wie oben beschrieben - verbessert die Lesbarkeit der Beschriftung deutlich.

In noch weiterer vorteilhafter Ausgestaltung bedeckt das Antirutsch-Element mehr als 60%, vorzugsweise mehr als 80% der Fläche des Fingerflansches. Hierdurch wird eine ausreichende bis besonders gute Abrutschsicherheit gewährleistet.

Darüber hinaus weist die Oberfläche vorteilhafterweise eine regelmäßige geometrische Struktur, d.h. ein sich wiederholendes Muster, oder eine unregelmäßige statistische Struktur auf, d.h. eine zufällige Verteilung von erhabenen und vertieften Stellen.

Weiterhin ist das Antirutsch-Element vorteilhafterweise von der Mantelfläche des zylindrischen Hohlraums beabstandet. Mit anderen Worten: Die raue Oberfläche des Antirutsch-Elements erstreckt sich nicht bis zum zylindrischen Teil des Spritzenkörpers, sondern endet kurz (z.B. mehr als 1 mm) vorher und geht in eine glatte Oberfläche über, die sich dann bis zum zylindrischen Teil erstreckt und an diesen anschließt. Hierdurch wird erreicht, dass das Nest im Gussprozess Kontakt zur glatten Oberfläche hat, so dass Partikelbildung vermieden wird.

Vorteilhaft ist der Spritzenkörper, d.h. die den Hohlraum umgebende Wandung, der Konus und der Fingerflansch, einstückig ausgebildet, d.h. z.B. direkt im Spritzgussverfahren hergestellt. Gerade bei derartigen im Spritzgussverfahren hergestellten Spritzenkörpern tritt das eingangs dargestellte Problem der Einfallstellen besonders zu Tage. Bei Glasspritzen hingegen kann es von Vorteil sein, aufsteckbare separate Fingerflansche zu verwenden, welche auf die Wandung aufgesteckt werden, da sich nichtzylindersymmetrische Fingerflansche aus Glas schwierig herstellen lassen.

In vorteilhafter Ausgestaltung besteht der Spritzenkörper aus einem Kunststoff, insbesondere einem Cycloolefin-Polymer oder -Copolymer. Insbesondere derartige Spritzenkörper sind vorteilhaft einstückig ausgebildet.

Eine pharmazeutische Spritze, umfasst vorteilhafterweise einen beschriebenen Spritzenkörper und einen beweglichen, in dem zylindrischen Hohlraum angeordneten Kolben.

Die mit der Erfindung erzielten Vorteile bestehen insbesondere darin, dass durch die Verwendung einer rauen Oberfläche mit einem arithmetischen Mittenrauwert von mehr als 5 µm als Antirutsch-Element am Fingerflansch einer

Spritze einerseits eine besonders gute Griffigkeit, andererseits eine besonders glatte Oberfläche des Flansches auf der gegenüberliegenden Seite erreicht wird, so dass ein Verkippen bei der Lagerung vermieden wird. Zudem verbessert eine derartige Oberfläche die Lesbarkeit einer ggf. vorhandenen Beschriftung.

Im labormedizinischen oder klinischen Alltag werden häufig bei der Zubereitung und der Applikation entsprechender Präparate, insbesondere bei deren Injektion, Substanzen verwendet, welche ein hohes Kontaminationsrisiko für den jeweiligen Anwender und auch für den Patienten aufweisen.

Dies können beispielsweise ionisierende Strahlung abgebende oder biologisch aktive Subtanzen des zu applizierenden Präparats sein, welche auch für das medizinische Personal ein hohes Schädigungsrisiko in sich tragen, insbesondere dann wenn beispielsweise ein Spritzenkörper nicht sachgerecht gehandhabt wird, welches beispielsweise dann geschehen kann, wenn der Spritzenkörper während seiner Handhabung nicht exakt positioniert wird oder dieser, beispielsweise bei der Applikation von hochviskosen Fluiden einen erhöhten Kraftaufwand erfordert.

Kommt es in diesen Fällen bei der Handhabung der Spritze zu einem Abrutschen vom Spritzenkörper, kann eine derartige Substanz austreten und sowohl mit dem Patienten als auch dem medizinischen Personal in unerwünschter Weise in Kontakt treten.

Aber auch eine weniger korrekt positionierte Injektion kann für den jeweiligen Patienten dann abträglich sein, wenn beispielsweise bei einer intramuskulären Depot-Abgabe ein Gefäß, wie beispielsweise ein Blut oder Lymphe führendes Gefäß mit der abzugebenden Substanz beaufschlagt wird.

Diese Situation wird in der Regel dann zusätzlich noch erschwert, wenn beispielsweise während der vorstehend erwähnten Handhabung Schutzhandschuhe getragen werden.

Hier erweist sich die vorliegend offenbarte Spritze mit deren Spritzenkörper als äußerst hilfreich, denn durch das vorliegend offenbarte Antirutsch-Element mit seiner Oberfläche, die mit einem arithmetischen Mittenrauwert zwischen 0,8 µm und 150 µm ausgebildet ist, wird diese Handhabung deutlich verbessert.

Zum Einen wird die Wahrscheinlichkeit eines Abrutschen stark vermindert und zum Anderen der exaktere Umgang, beispielsweise bei der Positionierung der vorzunehmenden Injektion erheblich verbessert.

Diese Vorteile zeigen sich insbesondere bei der Verwendung der vorliegend offenbarten Spritze mit deren Spritzenkörper in einem medizinischen oder kosmetischen Verfahren, umfassend
Injizieren oder Applizieren eines medizinischen Präparats in der Oralchirurgie insbesondere die Injektion eines Anästhetikums im Mundraum eines Patienten und/oder
Injizieren oder Applizieren eines medizinischen Präparats in der Ophthalmologie, insbesondere zur Unterdrückung von Lidreflexen oder zur Weitstellung der Pupillen eines Patienten und/oder
Injizieren oder Applizieren eines medizinischen Präparats in der der Nephrologie, insbesondere zur Applikation von Marker-Substanzen zur Verfolgung fluiddynamischer Prozesse und/oder
Injizieren oder Applizieren eines medizinischen Präparats in der Nuklearmedizin zur Applikation radioaktiv emittierender Isotope, vorzugsweise zur Tumorbehandlung, und/oder
Injizieren oder Applizieren eines kosmetischen Präparats, wie beispielsweise von Hyaluronsäure oder eines Botox-basierten Präparates.

Noch stärker ausgeprägt sind diese Vorteile, wenn beispielsweise ein Fluid injiziert oder appliziert wird, welches mit Schutzhandschuhen appliziert wird, insbesondere mit Schutzhandschuhen zur einmaligen Verwendung, welche DIN EN 455 "Medizinische Handschuhe zum einmaligen Gebrauch" entsprechen.

Hierbei kann beispielsweise ein Fluid injiziert oder appliziert werden, welches
ein patentenindividualisiertes Präparat umfasst, welches eine gentechnisch veränderte Substanz enthält, und/oder
ein biologisch oder mikrobiologisch hergestelltes oder vermehrtes Präparat umfasst und/oder
eine patientenindividualisierte tumorspezifische Markersubstanz umfasst.

Eine weitere vorteilhafte Verwendung der Spritze mit deren Spritzenkörper liegt vor bei einem Verfahren, bei welchem ein Fluid injiziert oder appliziert wird, welches mit Schutzhandschuhen, insbesondere mit Schutzhandschuhen gegen ionisierende Strahlen und radioaktive Kontamination, insbesondere mit Schutzhandschuhen entsprechend der DIN EN 421 appliziert wird.

Hierbei kann beispielsweiser ein Fluid mit deutlich erhöhter Sicherheit injiziert oder appliziert werden, welches ionisierende Strahlung erzeugt, welches insbesondere eine radioaktive patientenindividualiserte Tumormarkersubstanz und/oder ein radioaktive patientenidividualisierte Gensequenzen enthaltendes Präparat umfasst.

Obwohl kommerzielle Aspekte nicht im unmittelbaren Vordergrund der angestrebten Verbesserungen stehen, sei dennoch um der Vollständigkeit Willen angemerkt, dass die vorstehend erwähnten Präparate, insbesondere patientenindividualisierte Präparate sehr erhebliche Kosten bei deren Erstellung erzeugen können, welche insbesondere dann zu Tage treten, wenn eine nicht korrekt vorgenommene Applikation zu deren zumindest teilweisem Verlust führt.

Auch die Häufigkeit derartiger Fälle kann mit dem vorliegend offenbarten Verfahren vermindert werden.

Ausführungsbeispiele der Erfindung werden anhand von Zeichnungen näher erläutert. Darin zeigen
FIG 1 eine schematische Darstellung einer pharmazeutischen Spritze,
FIG 2 ein Antirutsch-Element am Fingerflansch der Spritze in dreidimensionaler Darstellung, und FIG 3 ein verbessertes Antirutsch-Element mit Darstellungen verschiedener rauer Oberflächen.

Gleiche Teile sind in allen Zeichnungen mit denselben Bezugszeichen versehen.

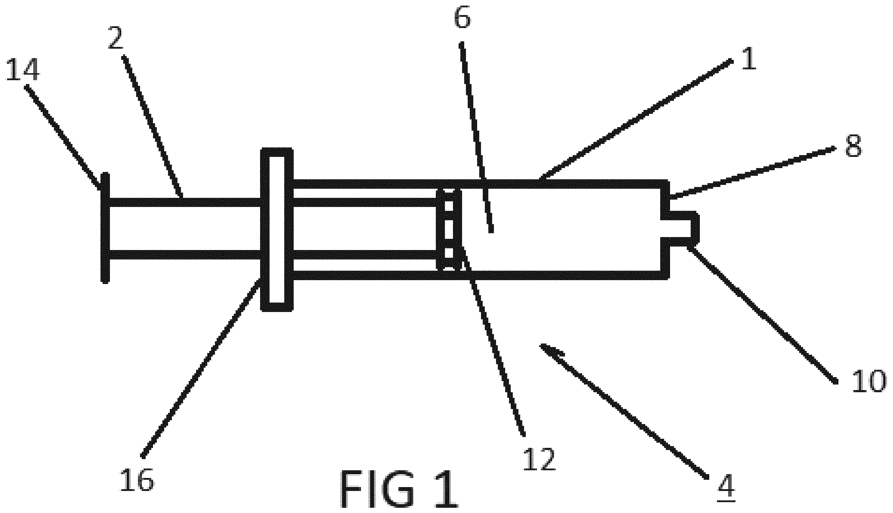

Die FIG 1 zeigt eine schematische Darstellung eines Spritzenkörpers 1 mit einem Kolben 2, die zusammen eine pharmazeutische Spritze 4 bilden. Der Spritzenkörper 1 ist aus einem transparenten Kunststoff gefertigt, in Ausführungsbeispielen einem Cycloolefin-Polymer oder - Copolymer. Er umfasst einen Hohlraum 6 mit kreiszylindrischer Wandung. Diese ist an einer ersten Grundfläche 8 im Wesentlichen verschlossen, allerdings kann dort durch einen Konus 10 die im Hohlraum 6 befindliche Flüssigkeit herausgedrückt werden. Dies erfolgt durch Hineindrücken des Kolbens 2, so dass der Kolbenboden 12, der kreisförmig ist und bündig in der Wandung des Hohlraums 6 anliegt, Druck auf die Flüssigkeit ausübt.

Zum Einführen des Kolbens 2 in den Hohlraum 6 ist dieser an der zweiten Grundfläche des Zylinders offen. Zum Hineindrücken des Kolbens 2 weist dieser auf der dem Kolbenboden 12 gegenüberliegenden Seite eine Druckfläche 14 auf, die typischerweise mit dem Daumen des Benutzers betätigt wird. Zum Gegenhalten ist um die kreisrunde Öffnung des Hohlraums 6 herum ein kragenförmiger Fingerflansch 16 angegossen. Dieser bildet um die Öffnung herum eine ebene Fläche, deren Normale die Achse des zylinderförmigen Hohlraums 6 ist.

Im Ausführungsbeispiel sind die Wandung des Hohlraums 6, der Konus 10 und der Fingerflansch 16 einstückig ausgeführt. Da der Spritzenkörper 1 im Ausführungsbeispiel aus einem Kunststoff gefertigt ist, kann dies z.B. dadurch realisiert sein, dass der Spritzenkörper 1 im Spritzgussverfahren hergestellt wurde. In einer alternativen Ausgestaltung kann die Wandung und der Konus 10 jedoch aus Glas gefertigt sein. In diesem Fall ist der Fingerflansch 16 als separates, aufsteckbares Teil ausgebildet.

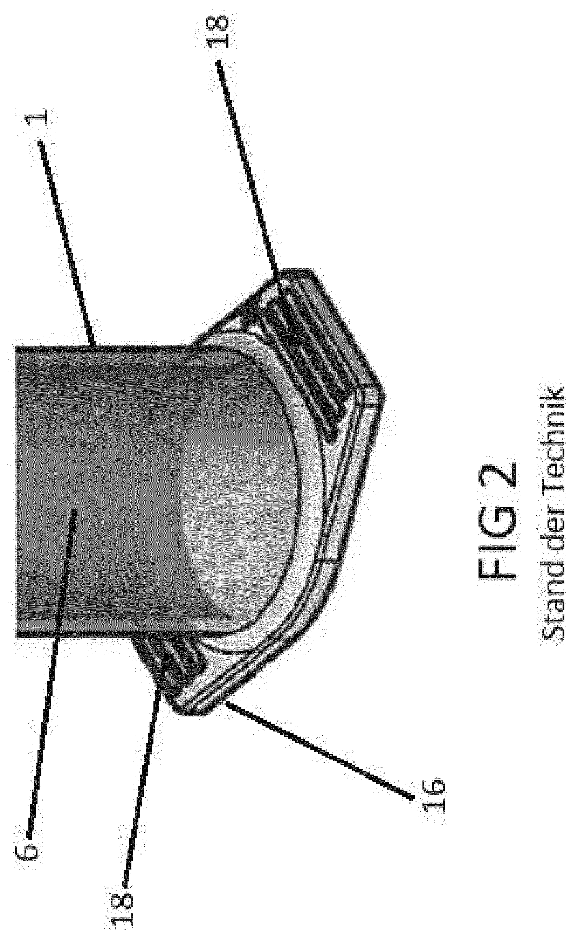

Die Gestaltung des Fingerflansches 16 wird im Folgenden näher beschrieben. FIG 2 zeigt eine dreidimensionale, teilweise abgeschnittene Darstellung eines Spritzenkörpers 1 im Bereich des Fingerflansches 16 gemäß dem Stand der Technik. Der Fingerflansch 16 ist bezüglich einer Ebene durch die Achse des zylinderförmigen Hohlraums 6 spiegelsymmetrisch aufgebaut. Er weist auf jeder der symmetrischen Seiten eine trapezförmige Außenkontur auf, wobei die lange Grundfläche des Trapezes nur geringfügig größer als der Außendurchmesser des Zylinders ist.

Hierdurch bilden sich zwei Flügel des Fingerflansches 16, von denen einer vom Zeigefinger, der andere vom Mittelfinger des Benutzers gegriffen werden kann, während der Daumen wie oben beschrieben den Kolben 2 niederdrückt. Um hierbei ein Abrutschen von Zeige- und Mittelfinger zu vermeiden, sind auf jedem der Flügel des Fingerflansches 16 Antirutsch-Elemente 18 auf der dem Konus 10 zugewandten Seite angeordnet. Diese sind im Stand der Technik nach FIG 2 als je drei deutlich erhabene Querstreben ausgebildet, die sich parallel zur Grundfläche des Trapezes des Flügels erstrecken.

Hierbei ergibt sich das Problem, dass der Spritzenkörper 1 in der in FIG 1 gezeigten Position, wenn er nämlich auf dem Fingerflansch 16 steht, nicht stabil ist, sondern verkippt.

Ursache sind Einsackungen auf der den Querstreben gegenüberliegenden Seite des Fingerflansches. Zudem behindern die Querstreben das Lesen einer auf der gegenüberliegenden des Fingerflansches 16 angeordneten Schrift.

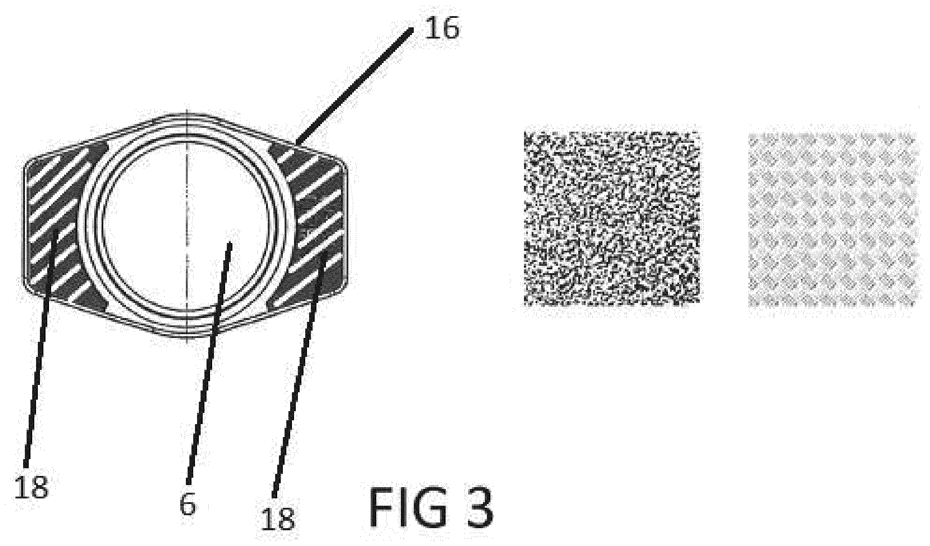

Zur Lösung dieser Probleme werden die Antirutsch-Elemente 18 wie in FIG 3 dargestellt ausgebildet, die nur anhand ihrer Unterschiede zu FIG 2 beschrieben wird. FIG 3 zeigt in der linken Bildhälfte den Fingerflansch 16 in der Aufsicht. Die Querstreben der Antirutsch-Elemente 18 sind entfallen.

Stattdessen ist in dem schraffierten Bereich, der mehr als 80% des Fingerflansches 16 ausmacht, die Oberfläche aufgeraut ausgeführt. Hierbei ist im bevorzugten Ausführungsbeispiel ein arithmetischer Mittenrauwert zwischen 10 und 50 µm vorgesehen. Zur Ermittlung dieses Messwertes wird die Oberfläche auf einer definierten Messstrecke abgetastet und sämtliche Höhen- und Tiefenunterschiede der rauen Oberfläche aufgezeichnet. Nach der Berechnung des bestimmten Integrals dieses Rauheitsverlaufes auf der Messstrecke wird abschließend dieses Ergebnis durch die Länge der Messstrecke dividiert, um den arithmetischen Mittenrauwert zu erhalten.

Die aufgeraute Oberfläche entsteht durch entsprechende Ausbildung der Gussform. Die rechte Bildhälfte der FIG 3 zeigt beispielhafte Möglichkeiten der Ausbildung der rauhen Oberfläche des Antirutsch-Element, so z.B. durch eine chaotische, statistische Aufrauhung der Oberfläche (links), oder aber ein regelmäßiges geometrisches Muster (rechts).

Zudem ist in FIG 3 in der linken Bildhälfte erkennbar, dass die schraffierte Fläche des so ausgestalteten Antirutsch-Elements 18 nicht bis zum Außendurchmesser des Hohlraums 6 heranreicht, sondern ein glatter Übergangsbereich zwischen Antirutsch-Element 18 und Mantelfläche des Hohlraums 6 verbleibt.

### Bezugszeichenliste

1 Spritzenkörper
2 Kolben
4 Spritze
6 Hohlraum
8 Grundfläche
10 Konus
12 Kolbenboden
14 Druckfläche
16 Fingerflansch
18 Antirutsch-Element

## Patentansprüche

1. Spritzenkörper (1) für eine pharmazeutische Spritze (4), umfassend einen zylindrischen Hohlraum (6) mit einer ersten Grundfläche (8) mit einem Konus (10),
einer zweiten, offenen Grundfläche zum Einführen eines Kolbens (2), und
mit einem im Bereich des Umfangs der offenen Grundfläche angeordneten Fingerflansch (16),
wobei der Fingerflansch (16) auf der dem Konus (10) zugewandten Seite ein Antirutsch-Element (18) zum Verhindern des Abrutschens eines Fingers beim Injektionsvorgang aufweist, wobei der
Fingerflansch (16) mit der offenen Grundfläche eine Ebene bildet und wobei der Fingerflansch (16) auf der dem Konus (10) abgewandten Seite glatt ausgebildet ist,
**dadurch gekennzeichnet, dass** das Antirutsch-Element (18) als Oberfläche mit
einem arithmetischen Mittenrauwert zwischen 5 µm und 50 µm ausgebildet ist, wobei der Spritzenkörper (1) einstückig ausgebildet ist.

2. Spritzenkörper (1) nach Anspruch 1, bei dem das Antirutsch-Element (18) als Oberfläche mit einem arithmetischen Mittenrauwert zwischen 10 µm und 50 µm ausgebildet ist.

3. Spritzenkörper (1) nach einem der vorhergehenden Ansprüche, bei dem der Fingerflansch (16) bezüglich einer die Achse des zylindrischen Hohlraums (6) enthaltenden Fläche spiegelsymmetrisch ausgebildet ist.

4. Spritzenkörper (1) nach einem der vorhergehenden Ansprüche, bei dem auf der dem Konus (10) abgewandten Seite des Fingerflansches (16) eine Beschriftungsfläche angeordnet ist.

5. Spritzenkörper (1) nach einem der vorhergehenden Ansprüche, bei dem das Antirutsch-Element (18) mehr als 60%, vorzugsweise mehr als 80% der Fläche des Fingerflansches (16) bedeckt.

6. Spritzenkörper (1) nach einem der vorhergehenden Ansprüche, bei dem die Oberfläche eine regelmäßige geometrische Struktur oder eine unregelmäßige statistische Struktur aufweist.

7. Spritzenkörper (1) nach einem der vorhergehenden Ansprüche, bei dem das Antirutsch-Element (18) von der Mantelfläche des zylindrischen Hohlraums (6) beabstandet ist.

8. Spritzenkörper (1) nach einem der vorhergehenden Ansprüche, der aus einem Kunststoff, insbesondere einem Cycloolefin-Polymer oder -Copolymer besteht.

9. Pharmazeutische oder kosmetische Spritze (4), umfassend einen Spritzenkörper (1) nach einem der vorhergehenden Ansprüche, und einen beweglichen, in dem zylindrischen Hohlraum (6) angeordneten Kolben (2).

10. Spritze nach Anspruch 9 umfassend ein Fluid zum Injizieren oder Applizieren, welches mit Schutzhandschuhen appliziert wird, insbesondere mit Schutzhandschuhen zur einmaligen Verwendung, welche DIN EN 455 "Medizinische Handschuhe zum einmaligen Gebrauch" entsprechen.

11. Spritze nach Anspruch 9 umfassend ein Fluid zum Injizieren oder Applizieren , welches
a. ein patientenindividualisiertes Präparat umfasst, welches eine gentechnisch veränderte Substanz enthält, oder
b. ein biologisch oder mikrobiologisch hergestelltes oder vermehrtes Präparat umfasst oder
eine patientenindividualisierte tumorspezifische Markersubstanz umfasst.

12. Spritze nach Anspruch 9 umfassend ein Fluid zum Injizieren oder Applizieren, welches mit Schutzhandschuhen gegen ionisierende Strahlen und radioaktive Kontamination, insbesondere mit Schutzhandschuhen entsprechend der DIN EN 421 appliziert wird.

13. Spritze nach Anspruch 9 umfassend ein Fluid zum Injizieren oder Applizieren, welches ionisierende Strahlung erzeugt.

14. Spritze nach Anspruch 9 umfassend ein Fluid zum Injizieren oder Applizieren, welches eine radioaktive patientenindividualiserte Tumormarkersubstanz oder ein radioaktive patientenidividualisierte Gensequenzen enthaltendes Präparat umfasst.

## Claims

1. A syringe body (1) for a pharmaceutical syringe (4), comprising a cylindrical cavity (6) which has
a first end face (8) with a cone (10);
a second, open end face for inserting a plunger (2); and
a finger flange (16) provided around the circumference of the open end face;
wherein the finger flange (16) has an anti-slip element (18) on the surface facing the cone (10), for preventing a finger from slipping off during an injection process;
wherein said finger flange (16) defines a plane together with the open end face, and wherein the finger flange (16) is smooth on the surface of the finger flange (16) facing away from the cone (10);
**characterized in that** the anti-slip element (18) as a surface exhibits an arithmetic mean roughness value between 5 µm and 50 µm, wherein the syringe body (1) is formed in one piece.

2. The syringe body (1) according to claim 1, wherein the anti-slip element (18) as a surface exhibits an arithmetic mean roughness value between 10 µm and 50 µm.

3. The syringe body (1) according to any one of the preceding claims, wherein the finger flange (16) has a mirror-symmetrical shape with respect to a surface containing the axis of the cylindrical cavity (6).

4. The syringe body (1) according to any one of the preceding claims, wherein a labelling area is provided on the surface of the finger flange (16) facing away from the cone (10).

5. The syringe body (1) according to any one of the preceding claims, wherein the anti-slip element (18) covers more than 60 %, preferably more than 80 % of the surface of the finger flange (16).

6. The syringe body (1) according to any one of the preceding claims, wherein the surface has a regular geometric texture or an irregular random texture.

7. The syringe body (1) according to any one of the preceding claims, wherein the anti-slip element (18) is spaced apart from the lateral surface of the cylindrical cavity (6).

8. The syringe body (1) according to any one of the preceding claims, made of a plastics material, in particular a cyclic olefin polymer or cyclic olefin copolymer.

9. A pharmaceutical or cosmetic syringe (4), comprising a syringe body (1) according to any one of the preceding claims, and a movable plunger (2) disposed inside the cylindrical cavity (6).

10. The syringe according to claim 9, comprising a fluid for injection or application, which is applied with protective gloves, in particular with protective gloves for single use according to DIN EN 455 "Medical gloves for single use".

11. The syringe according to claim 9, comprising a fluid for injection or application which
a. comprises a patient-individualized preparation that contains a genetically modified substance; or
b. comprises a biologically or microbiologically produced or propagated preparation; or
comprises a patient-individualized tumour-specific marker substance.

12. The syringe according to claim 9, comprising a fluid for injection or application, which is applied with protective gloves against ionizing radiation and radioactive contamination, in particular with protective gloves according to DIN EN 421.

13. The syringe according to claim 9, comprising a fluid for injection or application, which generates ionizing radiation.

14. The syringe according to claim 9, comprising a fluid for injection or application, which contains a radioactive patient-individualized tumour marker substance or a radioactive preparation containing patient-individualized gene sequences.

## Revendications

1. Corps de seringue (1) destiné à une seringue pharmaceutique (4), comprenant un espace creux (6) cylindrique doté d'une première surface de base (8) avec un cône (10), d'une deuxième surface de base ouverte, destinée à introduire un piston (2), et
comportant une bride pour les doigts (16) disposée dans la région de la périphérie de la surface de base ouverte, la bride pour les doigts (16) présentant, sur la face tournée vers le cône (10), un élément antidérapant (18) destiné à empêcher qu'un doigt ne dérape lors de l'opération d'injection, la bride pour les doigts (16) formant un plan avec la surface de base ouverte, et la bride pour les doigts (16) étant réalisée de manière lisse sur la face éloignée du cône (10),
**caractérisé en ce que** l'élément antidérapant (18) est réalisé sous la forme d'une surface avec une valeur de rugosité moyenne arithmétique comprise entre 5 µm et 50 µm, le corps de seringue (1) étant réalisé d'une seule pièce.

2. Corps de seringue (1) selon la revendication 1, dans lequel l'élément antidérapant (18) est réalisé comme surface ayant une valeur de rugosité moyenne arithmétique qui est comprise entre 10 µm et 50 µm.

3. Corps de seringue (1) selon une des revendications précédentes, dans lequel la bride pour les doigts (16) est réalisée de manière symétrique par réflexion par rapport à une surface contenant l'axe de l'espace creux (6) cylindrique.

4. Corps de seringue (1) selon une des revendications précédentes, dans lequel une surface d'inscription est disposée sur la face de la bride pour les doigts (16) qui est éloignée du cône (10).

5. Corps de seringue (1) selon une des revendications précédentes, dans lequel l'élément antidérapant (18) recouvre plus de 60 %, de préférence plus de 80 % de la surface de la bride pour les doigts (16).

6. Corps de seringue (1) selon une des revendications précédentes, dans lequel la surface présente une structure géométrique régulière ou une structure statistique irrégulière.

7. Corps de seringue (1) selon une des revendications précédentes, dans lequel l'élément antidérapant (18) se trouve à distance de la surface enveloppe de l'espace creux (6) cylindrique.

8. Corps de seringue (1) selon une des revendications précédentes, qui est constitué d'une matière plastique, en particulier d'un polymère ou d'un copolymère d'oléfine cyclique.

9. Seringue (4) pharmaceutique ou cosmétique, comprenant un corps de seringue (1) selon une des revendications précédentes, et un piston (2) mobile disposé dans l'espace creux (6) cylindrique.

10. Seringue selon la revendication 9, comprenant un fluide destiné à l'injection ou à l'application, qui est appliqué avec des gants de protection, notamment avec des gants de protection à usage unique conformes à DIN EN 455 « gants médicaux à usage unique ».

11. Seringue selon la revendication 9, comprenant un fluide destiné à l'injection ou à l'application, qui
a. comprend un produit individualisé en fonction du patient, lequel contient une substance génétiquement modifiée, ou
b. comprend un produit fabriqué ou reproduit par voie biologique ou microbiologique ou
comprend une substance de marqueur spécifique à une tumeur et individualisée en fonction du patient.

12. Seringue selon la revendication 9, comprenant un fluide destiné à l'injection ou à l'application, qui est appliqué avec des gants de protection contre les rayonnements ionisants et la contamination radioactive, notamment avec des gants de protection conformes à DIN EN 421.

13. Seringue selon la revendication 9, comprenant un fluide destiné à l'injection ou à l'application, qui génère un rayonnement ionisant.

14. Seringue selon la revendication 9, comprenant un fluide destiné à l'injection ou à l'application, qui comprend une substance de marqueur tumoral radioactive, individualisée en fonction du patient, ou un produit contenant des séquences de gènes radioactives individualisées en fonction du patient.
